# EUROPÄISCHE PATENTSCHRIFT

(11) **EP 2 497 432 B1**
(45) Veröffentlichungstag und Bekanntmachung des Hinweises auf die Patenterteilung: **20.08.2014**
(21) Anmeldenummer: 12158066.6
(22) Anmeldetag: 05.03.2012
(51) Int. Cl.: A61B 17/16

(54) **Medizinisches Einstechwerkzeug**
Medical punching tool
Outil d'insertion médical

(30) Priorität: 07.03.2011 DE 102011013888
(43) Veröffentlichungstag der Anmeldung: 12.09.2012
(73) Patentinhaber: Karl Storz GmbH & Co. KG, 78532 Tuttlingen (DE)
(72) Erfinder: Berberich, Sascha, 78532 Tuttlingen (DE); Frey, Sebastian, 68753 Waghäusel (DE)
(74) Vertreter: Witte, Weller & Partner Patentanwälte mbB

(56) Entgegenhaltungen:
- WO-A1-2011/014677
- US-A1- 2001 021 853
- US-A1- 2006 074 427
- US-A1- 2006 074 428

## Beschreibung

Die Erfindung betrifft ein medizinisches Einstechwerkzeug zum Eindringen in einen Knochen, mit einem eine Längsachse aufweisenden Schaft, an dessen distalem Ende ein Werkzeug in Form einer Stanze oder eines Multi-Mikrofrakturiermeißels angeordnet ist.

Ein medizinisches Einstechwerkzeug in Form einer Knorpelstanze ist beispielsweise aus der DE 10 2005 010 988 A1 bekannt.

Mit einer solchen Stanze kann an einer Knochenoberfläche ein Bereich der knorpelartigen Knochenhaut ausgestanzt werden. Dies erfolgt dann, wenn diese Knochenhaut beispielsweise einen Defekt aufweist und diese Defektstelle repariert werden soll, beispielsweise durch Einbringen eines entsprechenden Implantates.

Derartige Defektstellen treten insbesondere in Gelenken auf, an denen zwei gegenüberliegende Knochen aneinander reiben. Bei gesunden Gelenken befindet sich zwischen den Knochen eine entsprechende Knorpelschicht und auch eine Knorpelflüssigkeit, die für eine Schmierung der Bewegung der beiden Knochen aneinander sorgt.

Ist diese Gelenkflüssigkeit nicht mehr vorhanden, sei es aufgrund von Verletzungen oder manchmal auch aufgrund von Alterserscheinungen, reiben die Knochen direkt über ihre Knorpel bzw. über deren äußere Knochenhaut aneinander. Dabei entstehen dann an den Berührungsstellen sehr schmerzhafte Defekte.

Zur Behebung des Defektes wird zunächst mit einem Einstechwerkzeug in Form einer Stanze, die eine distalseitige Schneide aufweist, ein flächiges Stück der Knochenhaut im Bereich des Defektes ausgestanzt. Dabei ist der Schaft des Einstechwerkzeuges hohl ausgebildet, so dass durch den Schaft hindurch das ausgestanzte Knochenhautstück entfernt werden kann. Für den späteren Erfolg eines Anwachsens eines Implantates ist sehr wichtig, dass der Schnitt durch die umlaufende Schneide mit einer möglichst geraden Schnittkante erfolgt, insbesondere mit einer Schnittkante, die senkrecht zur durch die Schneide beschriebenen Fläche verläuft.

Ein medizinisches Einstechwerkzeug in Form eines Mikrofrakturiermeißels ist aus der DE 10 2005 010 989 A1 bekannt.

Dieses Einstechwerkzeug weist mehrere vom distalen Ende vorspringende Zapfen oder Spitzen auf. Mit diesen sollen Mikrofrakturen in einen Knochen eingebracht werden. Durch diese Mikrofrakturen kann ein Gerinnsel, das mit pluripotenten Stammzellen aus dem Knochenmark durchsetzt wird, erzeugt werden. Ein solches Gerinnsel wird auch manchmal als "Super Clot" bezeichnet. Die in diesem Gerinnsel enthaltenen Stammzellen können sich im Laufe des Heilungsprozesses zu Knorpelzellen differenzieren und neues Knorpelgewebe bilden. Das heißt, diese Multi-Mikrofrakturiermeißel können ebenfalls bei der Reparatur von Knorpeldefekten an Knochen eingesetzt werden.

So kann beispielsweise, nachdem mit der eingangs erwähnten Stanze die Knochenhaut im Bereich der Defektstelle abgetragen wurde, mit einem Multi-Mikrofrakturiermeißel ein solches Gerinnsel hervorgerufen werden, das dann an der ausgestanzten Stelle den Knorpel ersetzt.

Es wurde auch festgestellt, dass das Anwachsen eines Implantates, das an die ausgestanzte Stelle eingesetzt wird, durch solche Gerinnsel erheblich beschleunigt wird.

Beiden Einstechwerkzeugen ist gemein, dass bei der Handhabung eine längs der Längsachse des Schaftes ausgeübte Kraft eingesetzt werden muss, um entweder die Schneide der Stanze in das Knorpelgewebe einzudrücken oder den Multi-Mikrofrakturiermeißel in den Knochen einzuschlagen.

Es ist aber nun so, dass solche Defektstellen meist relativ weit innen im Bereich von Gelenken auftreten, insbesondere im Bereich des Kniegelenkes, also an einer für einen sich geradlinig erstreckten steifen Schaft relativ schwer zugänglichen Stelle. Man muss dann das Gelenk extrem verbiegen oder spreizen, um an solche Stellen heranzukommen.

Aus der US 2006/0074427 A1 ist ein medizinisches Einstechwerkzeug zum Eindringen in einen Knochen bekannt, das einen eine Längsachse aufweisenden Schaft zeigt, an dessen distalem Ende ein Werkzeugträger angeordnet ist, an dessen distalem Ende ein Werkzeug vorhanden ist.

Es ist ein Verstellmechanismus vorhanden, über den die Winkelstellung zwischen Schaft und Werkzeugträger verändert werden kann. Dieser Verstellmechanismus weist eine im Inneren des Schafts angeordnete, axial bewegliche Stange auf, über die der Werkzeugträger zum Verstellen etwas vom distalen Ende des Schafts abgehoben werden kann. In diesem Zustand kann der Werkzeugträger um eine Schwenkachse verschwenkt werden. Danach wird über die Schubstange der verschwenkte Werkzeugträger wieder an das distale Ende des Schaftes heranbewegt, wodurch er dann in einer festen, nicht mehr veränderbaren Stellung verbleibt.

Dabei ist am Werkzeugträger eine mit Zähnen versehene Scheibe vorhanden, die in entsprechende Ausnehmungen am distalen Ende des Schaftes sperrend eintreten können. Dadurch ist eine endliche Anzahl an Verschwenkstellungen zwischen dem Schaft und dem Werkzeugträger möglich. Im praktischen Einsatz ist die Verbindung zwischen Schaft und Werkzeugträger starr, das heißt nicht verschwenkbar.

Es ist daher Aufgabe der vorliegenden Erfindung, hier Abhilfe zu schaffen und ein Einstechwerkzeug der eingangs genannten Art dahingehend zu verbessern, dass auch relativ schwer zugängliche Stellen in Gelenken erreicht werden können, um dort eine Defektstelle zu reparieren.

Erfindungsgemäß wird die Aufgabe dadurch gelöst, dass das Werkzeug an einem distalseitigen Werkzeugträger angeordnet ist, der, ohne Zuhilfenahme weiterer Hilfs- oder Steuermittel, frei schwenkbar am distalen Ende des Schaftes angebracht ist, wobei ein Schwenkwinkel der Verschwenkbarkeit des Werkzeugträgers aus der Längsachse des Schaftes heraus durch einen Anschlag auf maximal 45° begrenzt ist.

Durch die Verschwenkbarkeit des Werkzeugträgers kann das Einstechwerkzeug mit einem zur zu behandelnden Knochenfläche schräg bzw. gekippten Schaft angezielt werden. Durch den frei schwenkbaren Werkzeugträger kann das Werkzeug einfach so ausgerichtet werden, dass dessen distale Endseite mit dem Werkzeug etwa parallel zu der Oberfläche zum Liegen kommt, in die eingestochen werden soll.

Dadurch kann das eigentliche Werkzeug, also entweder die Stanze mit der umlaufenden Schneide oder die distal vorspringenden Stifte des Multi-Mikrofrakturiermeißels, in einer Ausrichtung in die Knochenhaut eingetrieben werden, die für ein optimales Ergebnis sorgen. Wie erwähnt, wird ein optimales Ergebnis beim Stanzen dann erzielt, wenn die Schneide der Stanze, die meist kreisförmig umlaufend ist, in einer Ausrichtung eingedrückt wird, bei der die durch die Stanze umgrenzte Ebene parallel zur Knochenfläche liegt. Das erzeugt dann an der umfänglichen verbleibenden Knorpelwand eine definierte etwa rechtwinklig von der Knochenoberfläche hochstehende umlaufende Kante des verbleibenden Knochenhautgewebes.

Gleiches gilt für den Multi-Mikrofrakturiermeißel. Auch hier wird ein bestes Ergebnis erzielt, wenn die Stifte des Meißels in einer Richtung senkrecht zur Knochenoberfläche eingetrieben werden.

Durch die Verschwenkbarkeit des Werkzeugträgers ist es nun möglich, an relativ unzugänglichen Stellen das Einstechwerkzeug zu benutzen und dabei das eigentliche Werkzeug so zur Knochenoberfläche auszurichten, wie wenn das ein nicht schwenkbarer starrer geradliniger Schaft wäre.

Durch die Vorsehung eines Anschlages, der die Verschwenkbewegung auf maximal 45° begrenzt, ist es möglich durch eine axiale Vorschubbewegung, die in Richtung der Längsachse des Schaftes ausgeübt wird, noch die notwendige Kraftkomponente zum Eindrücken oder Einschlagen des Werkzeuges in einer Richtung senkrecht zur Knochenoberfläche aufzubringen.

In anderen Worten ausgedrückt kann die Handhabungsperson wie bisher die Kraft zum Einstechen des Werkzeuges, also entweder zum Eindrücken oder zum Einschlagen, wie gewohnt ausüben, da durch den maximal auf 45° begrenzten Winkel ein ausreichend großer Teil dieser Kraft in die gewünschte Richtung senkrecht zur Knochenfläche ausgeübt wird.

Durch die erfindungsgemäße Ausgestaltung ist ein erheblich erweiterter Bereich dieser Reparaturtechnik zu erzielen, indem an wesentlich weiter innen liegenden Knochenflächen solche Defekte repariert werden können, ohne dass dabei die beiden Knochen extrem zueinander bewegt oder voneinander weggezogen werden müssen.

Somit ist letztendlich auch ein solcher Eingriff für einen Patienten wesentlich atraumatischer durchzuführen.

In einer Ausgestaltung der Erfindung ist der Schwenkwinkel auf maximal 30° begrenzt.

Diese Maßnahme hat den Vorteil, dass durch diese Winkelbegrenzung ein großer Teil der in Richtung der Längsachse des Schaftes ausgeübten Kräfte beim Einstechen auch tatsächlich über das Werkzeug senkrecht zur Knochenoberfläche abgeleitet werden können. Diese Ausgestaltung ist besonders vorteilhaft, wenn Knorpeldefekte an relativ weit außen liegenden Stellen eines Gelenkes, insbesondere im umfänglichen Bereich des Kniegelenkes durchgeführt werden sollen.

In einer weiteren Ausgestaltung der Erfindung ist der Schwenkwinkel durch den Anschlag auf maximal 15° begrenzt.

Diese Ausgestaltung hat den Vorteil, dass nahezu die vollständige Eintreibkraft auf das Werkzeug übertragen werden kann.

Diese Ausgestaltung wird dann eingesetzt werden, wenn weit außen liegende Bereiche eines Gelenkes mit dem Einstechwerkzeug bearbeitet werden sollen und es notwendig ist, hohe Kräfte zu übertragen. Dadurch, dass der überwiegende Teil der ausgeübten Kraft über das Werkzeug in den Knochen abgeleitet wird, braucht man keine solch hohen Kräfte auszuüben, wie bei stärker abgewinkelten Werkzeugträgern, bei denen die Tendenz besteht, dass bei starken Krafteinwirkungen der Werkzeugträger seitlich versetzt oder abkippt.

In einer weiteren Ausgestaltung der Erfindung weist der Werkzeugträger eine axiale Länge von maximal 30 mm, insbesondere 20 mm oder höchst vorzugsweise von nur 10 mm auf.

Diese Ausgestaltungen haben den Vorteil, dass durch die geringe axiale Länge des Werkzeugträgers nur ein relativ geringer Raum zwischen zwei gegenüberliegenden Knochen benötigt wird, um das Werkzeug zwischen diese einzuschieben und den Einstechvorgang durchzuführen. Im Kniegelenk können solche Spaltbreiten durch ein extremes Abwinkeln von dem Unterschenkel gegenüber dem Oberschenkel erzielt werden, so dass ohne zusätzliche Spreizmaßnahmen schon relativ weit innen im Gelenk Defekte bearbeitet werden können.

In einer weiteren Ausgestaltung der Erfindung ist der Werkzeugträger um einen sich quer zur Längsachse erstreckenden Achsbolzen frei verschwenkbar.

Diese Maßnahme hat nicht nur den Vorteil, dass die gelenkige, somit verschwenkbare Verbindung zwischen Schaft und Werkzeugträger durch mechanisch sehr einfache Mittel zu bewerkstelligen ist, sondern dass durch den Bolzen auch ein mechanisch stabiles Mittel geschaffen wird, dass die Kräfte, die auf den Schaft einwirken, auf den Werkzeugträger übertragen werden können.

Diese quer zur Längsachse resultierende Schwenkachse aufgrund des Achsbolzens gibt der Handhabungsperson auch eine klare Anleitung, in welche Richtung sich der Werkzeugträger verschwenken kann. Dies gibt ihm dann auch eine Orientierungshilfe, wie er das medizinische Einstechwerkzeug, sprich in welcher Drehausrichtung er es zwischen die beiden Knochen beispielsweise in einem Kniegelenk, einführen muss, um die gewünschte Ausrichtung entweder der Stanze oder des Multi-Mikrofrakturiermeißels zu erzielen.

In einer weiteren Ausgestaltung der Erfindung ist der Werkzeugträger über ein Kugelgelenk in allen Richtungen frei verschwenkbar abgebracht.

Diese Maßnahme hat den erheblichen Vorteil, dass der Werkzeugträger in allen Richtungen aus der Längsachse heraus frei verschwenkbar ist. Der Begriff "frei verschwenkbar" bedeutet, dass die Verschwenkbarkeit ohne Zuhilfenahme weiterer Hilfs- oder Steuermittel erzielt werden kann, so dass beispielsweise allein durch ein schräges Halten der Werkzeugträger um das Kugelgelenk aufgrund der Schwerkraft verschwenkt wird. Dasselbe gilt selbstverständlich auch für die Ausgestaltung mit dem Achsbolzen, wenn dieser entsprechend locker eingesetzt ist.

In einer weiteren Ausgestaltung der Erfindung weist der Anschlag eine schräge Anschlagfläche am distalen Ende des Schaftes aus.

Diese Maßnahme hat den Vorteil, dass durch mechanisch einfache und robuste Mittel eine Anschlagfläche gestaltet wird, die die Verschwenkbewegung des Werkzeugträgers innehält. Durch entsprechende Ausbildung des Anschlages kann dann bei ein und demselben Werkzeugträger mit entsprechend unterschiedlichen Schäften der maximale Verschwenkwinkel begrenzt werden, beispielsweise auf die zuvor erwähnten Werte unter 45°, beispielsweise maximal 30° oder maximal 15°.

Die Anschlagfläche bildet bei maximalem Verschwenkwinkel eine relativ große Kontaktfläche zwischen Werkzeugträger und Schaft, über die die notwendigen Kräfte beim Einstechen übertragen werden können.

In einer weiteren Ausgestaltung der Erfindung ist die Anschlagfläche derart ausgebildet, dass eine Verschwenkung aus der Längsachse nur nach einer Seite durchführbar ist.

Diese Maßnahme hat den Vorteil, dass der Werkzeugträger ganz definiert um eine Schwenkachse nur in einer einzigen Richtung aus der Längsachse verschwenkbar ist.

Dies ist beispielsweise dadurch zu erzielen, dass die distale Endfläche des Schaftes nur einseitig angeschrägt wird, so dass dem Werkzeugträger nur eine Schwenkbewegung in Richtung dieser Schräge ermöglicht ist. Die Winkelstellung dieser Schräge erlaubt die Einstellung der unterschiedlichen maximalen Schwenkwinkel. Der Anschlag als solcher enthält selbstverständlich auch eine Anschlagfläche am proximalen Ende des Werkzeugträgers, die beim Verschwenken desselben dann mit der entsprechenden Anschlagfläche am distalen Ende des Schaftes in Anlage kommt und somit die Schwenkbewegung begrenzt ist.

In einer weiteren Ausgestaltung der Erfindung ist der Werkzeugträger über eine Art Scharniergelenk mit einem hohlzylindrischen Schaft verbunden und der Körper des Werkzeugträgers ist ebenfalls hohlzylindrisch, wobei die Teile Pfanne und Gelenkkopf des Scharniergelenkes jeweils aus der hohlzylindrischen Wand von Schaft und hohlzylindrischem Werkzeugträger herausgearbeitet sind.

Diese Maßnahme hat den Vorteil, dass nahezu der gesamte Innenraum des hohlzylindrischen Schaftes bzw. des hohlzylindrischen Werkzeugträgers für Manipulationen zur Verfügung steht. Diese Ausgestaltung ist insbesondere bei der Ausgestaltung als Stanze vorteilhaft, denn durch diesen hohlen Innenraum soll das durch die Stanze ausgestanzte Knorpelstück mit einem Spatel abgetragen und entfernt werden. Die Bauteile Pfanne und Gelenkkopf des Scharniergelenkes sind aus den Wänden herausgearbeitet. Der Gelenkkopf kann nach proximal vom Werkzeugträger oder nach distal vom distalen Ende des hohlzylindrischen Schaftes vorstehen. Entsprechend ist dann die Pfanne in der gegenüberliegenden Zylinderwand des entsprechenden Bauteils ausgespart. Dies ist nach heutiger Technologie durch Laserschneiden oder dergleichen mit hoher Präzision durchzuführen und die Teile sind einfach aneinanderzufügen. Die Verschwenkbarkeit wird dann durch entsprechende Abschrägung, beispielsweise der distalseitigen Stirnkante der Wand des Schaftes oder der entsprechend gegenüberliegenden Seite des Werkzeugträgers oder durch beide erzielt, so dass dann variable Verschwenkwinkel und auch Verschwenkrichtungen eingestellt werden können.

Es versteht sich, dass die vorstehend genannten und die nachstehend noch zu erläuternden Merkmale nicht nur in der jeweils angegebenen Kombination, sondern auch in anderen Kombinationen einsetzbar sind, ohne den der vorliegenden Erfindung zu verlassen.

Die Erfindung wird nachfolgend anhand einiger ausgewählter Ausführungsbeispiele im Zusammenhang mit den beiliegenden Zeichnungen beschrieben und erläutert. Es zeigen:
- Figur 1: eine Seitenansicht eines ersten Ausführungsbeispiels eines Einstechwerkzeuges in Form einer Stanze,
- Figur 2: perspektivisch einen Längsschnitt durch das Werkzeug von Figur 1,
- Figur 3: eine stark vergrößere Seitenansicht des distalen Endbereiches des Einstechwerkzeuges mit abgewinkeltem Werkzeugträger,
- Figur 4: eine perspektivische Darstellung eines zweiten Ausführungsbeispiels in Form eines Multi-Mikrofrakturiermeißels,
- Figur 5: eine stark vergrößerte Seitenansicht des distalen Endbereiches des Einstechwerkzeuges von Figur 4 mit geradlinig ausgerichtetem Werkzeugträger,
- Figur 6: eine der Figur 5 entsprechende Seitenansicht mit maximal abgewinkeltem Werkzeugträger,
- Figur 7: eine perspektivische Darstellung eines dritten Ausführungsbeispiels eines Einstechwerkzeuges, bei dem der Werkzeugträger über ein Kugelgelenk mit dem Schaft verbunden ist,
- Figur 8: eine stark vergrößerte Seitenansicht des distalen Endbereiches des Einstechwerkzeuges von Figur 7,
- Figur 9: stark schematisch ein Anwendungsbeispiel anhand eines vierten Ausführungsbeispiels, das dem Ausführungsbeispiel von Figur 1 ähnelt in Form einer Knorpelstanze beim Einsatz bzw. Ansetzen an einer Knochenoberfläche des Unterschenkels im Bereich dessen Kniegelenkes, und
- Figur 10: die Endstellung des Einstechwerkzeuges mit zutreffend ausgerichteter Stanze vor Beginn des Eindrückens.

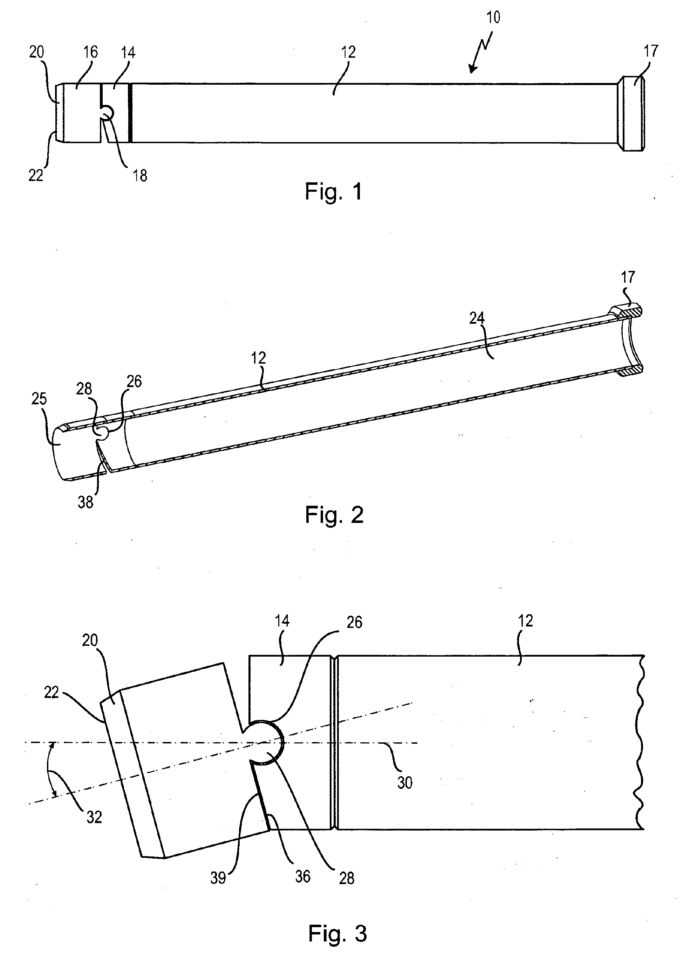

Ein in den Fig. 1 bis 3 dargestelltes erstes Ausführungsbeispiel eines erfindungsgemäßen Einstechwerkzeuges ist in seiner Gesamtheit mit der Bezugsziffer 10 bezeichnet.

Das Einstechwerkzeug 10 weist einen Schaft 12 auf, an dessen distalem Ende 14 ein Werkzeugträger 16 gelenkig, somit verschwenkbar, angebracht ist. Am proximalen Ende des Schaftes 12 ist ein Schlagkopf 17 vorgesehen, über den eine Kraft auf den Schaft ausgeübt werden kann, beispielsweise durch ein entsprechendes Eintreibwerkzeug, wie einen Hammer oder dergleichen.

Der Werkzeugträger 16 ist über ein Gelenk 18 mit dem Schaft 12 verbunden.

Das Werkzeug 20 des Werkzeugträgers 16 ist als eine keilförmig umlaufende Stanze mit einer Schneide 22 ausgebildet.

Sowohl der Schaft 12 ist als Hohlzylinder 24 als auch der Werkzeugträger 16 ist als ein Hohlzylinder 25 ausgebildet, wie das insbesondere aus der Schnittdarstellung von Fig. 2 ersichtlich ist.

Das Gelenk 18 ist in Art eines Scharniergelenkes ausgebildet. An diametral gegenüberliegenden Stellen am distalen Ende 14 des Schaftes 12 ist in der hier nicht näher bezeichneten Wand des Hohlzylinders jeweils eine Pfanne 26 ausgespart.

Vom proximalen Ende des Werkzeugträgers 16 stehen entsprechend ausgeformte Gelenkköpfe 28 vor, die ebenfalls aus der Wand des Hohlzylinders 25 herausgearbeitet worden sind. Ein Gelenkkopf 28 passt in eine Pfanne 26. Eine entsprechende Bemaßung verhindert ein seitliches Abgleiten der Bauteile Werkzeugträger 16 und Schaft 12 quer zur Längsachse 30 voneinander.

Der Werkzeugträger 16 ist somit aus der Längsachse 30 des Schaftes 12 um den Gelenkkopf 28 verschwenkbar, und zwar in einem Schwenkwinkel 32 von maximal 15°.

Ein Anschlag 36 begrenzt diese Schwenkbewegung, die nur in einer einzigen Richtung von der Längsachse 30 weggerichtet möglich ist, in der Darstellung von Fig. 3 nur nach unten.

Der Anschlag 36 besteht aus einer abgeschrägten Anschlagfläche 38 am distalen Ende 14 des hohlzylindrischen Schaftes 12. Diese geneigte Anschlagfläche 38 stellt also eine etwa halbkreisförmige abgeschrägte Stirnfläche des distalen Endes 14 des Schaftes 12 dar, die sich um einen Halbkreis zwischen den gegenüberliegenden Pfannen 26 erstreckt.

Die dieser Anschlagfläche 38 radial gegenüberliegende Stirnseite verläuft exakt rechtwinklig zur Längsachse 30.

Die proximalseitige Stirnfläche bzw. Endfläche 39 des hohlzylindrischen Werkzeugträgers 16 ist als umlaufende Kante ausgebildet, deren Ebene sich senkrecht zur Längsachse des Werkzeugträgers 16 erstreckt.

Somit liegt der Verschwenkbereich des Werkzeugträgers 16 zwischen der in Fig. 1 dargestellten geradlinigen Ausrichtung, also in Ausrichtung mit der Längsachse 30, und der um 15° in eine Richtung verschwenkten Position von Fig. 3.

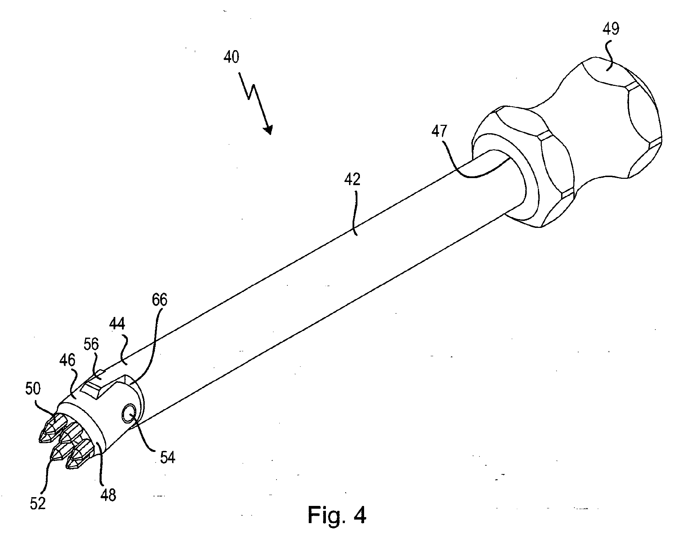

Ein in den Fig. 4 bis 6 dargestelltes zweites Ausführungsbeispiel eines erfindungsgemäßen Einstechwerkzeuges ist in seiner Gesamtheit mit dem Bezugszeichen 40 versehen.

Das Einstechwerkzeug 40 weist ebenfalls einen geradlinig verlaufenden lang erstreckten Schaft 42 auf, an dessen distalem Ende 44 ein Werkzeugträger 46 gelenkig angebracht ist.

Der Werkzeugträger 46 trägt ein Werkzeug in Form eines Multi-Mikrofrakturiermeißels 48. Dieser Meißel weist mehrere nach distal vorspringende Zapfen 50 auf, die jeweils in einer Spitze 52 enden. Proximalseitig ist der Schaft 12 mit einem Griff 49 verbunden.

Die gelenkige Verbindung zwischen Schaft 42 und Werkzeugträger 46 erfolgt über einen sich quer zur Längsachse 60 des Schaftes 42 erstreckenden Achsbolzen 54, der sich durch einen mittigen Steg 56, der distalseitig vom Schaft 42 vorspringt, und zwei hier nicht näher bezeichnete proximal vorstehende Gabelarme des Werkzeugträgers 46 hindurch erstreckt. Der Anschlag 64 wird durch entsprechende Anschlagflächen 66 am distalen Ende des Schaftes 42 gebildet, von dem der Steg 56 vorspringt. Diese Anschlagfläche 66 verläuft senkrecht zur Längsachse 60. An diese Anschlagfläche 66 schlägt dann eine etwa bogenförmig gekrümmte proximalseitige Endfläche des Werkzeugträgers 46 an.

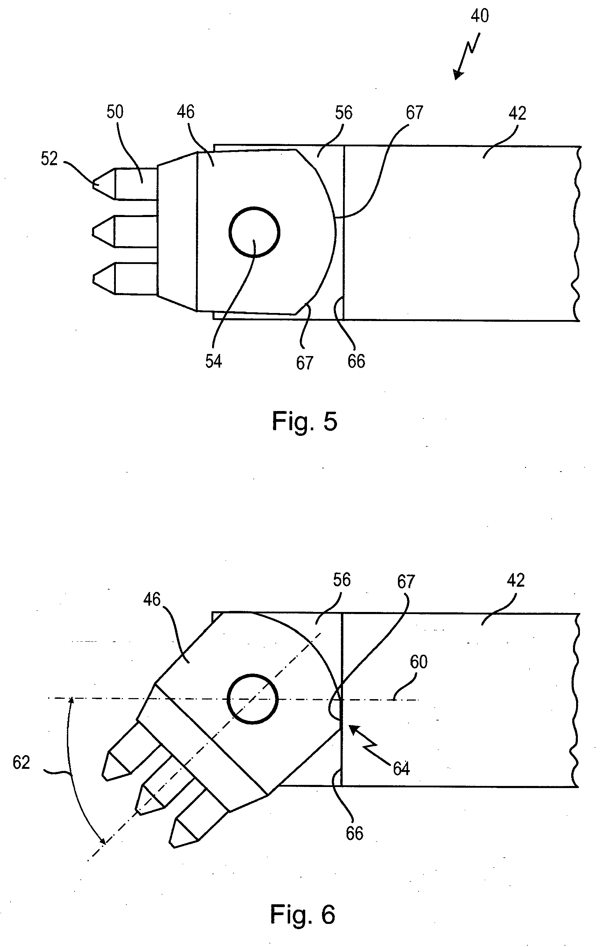

In Fig. 6 ist eine Situation dargestellt, wie der Werkzeugträger 46 um den Achsbolzen 54 um seinen maximalen Schwenkwinkel 62 von etwa 45° verschwenkt ist. In dieser maximalen Verschwenkstellung trifft die Endfläche 67 des Werkzeugträgers 46 auf die Anschlagfläche 66 am distalen Ende des Schaftes 42. Der Werkzeugträger 46 kann auch um den Schwenkwinkel 62 von etwa 45° in die entgegengesetzte Richtung maximal verschwenkt werden.

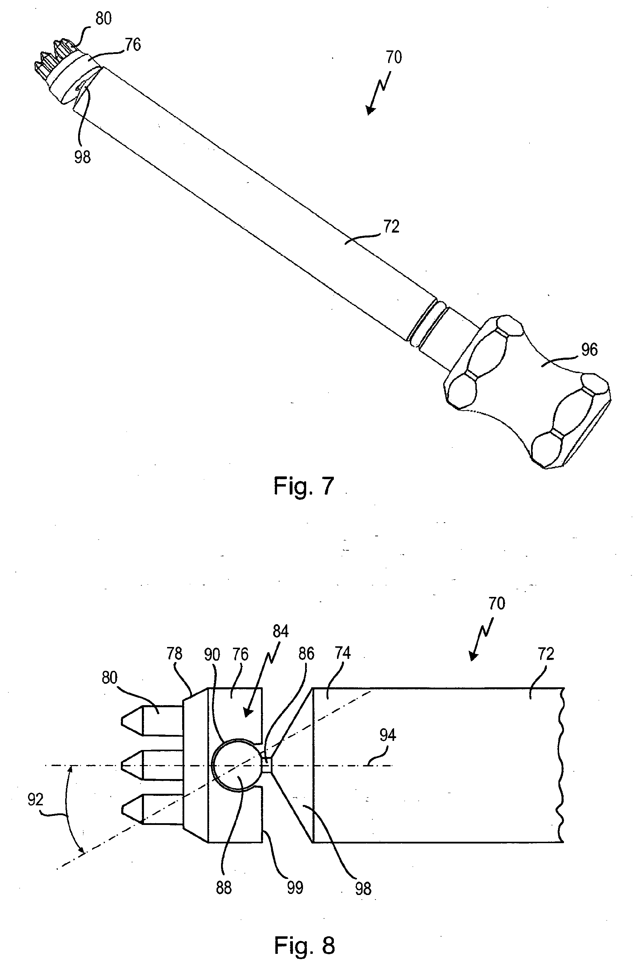

In den Fig. 7 und 8 ist ein drittes Ausführungsbeispiel eines erfindungsgemäßen Einstechwerkzeuges dargestellt, das in seiner Gesamtheit mit der Bezugsziffer 70 versehen ist.

Das Einstechwerkzeug 70 ist ähnlich wie das Einstechwerkzeug 40 ausgebildet und weist einen lang erstreckten Schaft 72 auf, an dessen distalem Ende ein Werkzeugträger 76 angebracht ist, dessen eigentliches Werkzeug als Multi-Mikrofrakturiermeißel 78 mit den entsprechend vorstehenden Zapfen 80 ausgebildet ist. Die gelenkige Verbindung zwischen Schaft 72 und Werkzeugträger 76 erfolgt hier über ein Kugelgelenk 84, wie das näher in Fig. 8 ersichtlich ist. Das Kugelgelenk 84 weist einen vom distalen Ende 74 des Schaftes 72 vorstehenden Zapfen 86 auf, der eine Kugel 88 trägt. Die Kugel 88 ist in einer Kugelpfanne 90 im Inneren des Werkzeugträgers 76 aufgenommen. Der Anschlag 98 wird durch eine kegelartig nach distal vorspringende Endfläche 98 des distalen Endes des Schaftes 72 gebildet. Diese Kegelfläche wirkt mit der proximalseitigen Endfläche 99 des Werkzeugträgers 76 zusammen, die sich in der in Fig. 8 dargestellten Ausrichtung senkrecht zur Längsachse 94 des Einstechwerkzeuges 70 erstreckt.

Der maximale Schwenkwinkel 92 beträgt hier 30°, wobei die Richtung der Verschwenkung aufgrund des Kugelgelenkes 84 in sämtlichen Richtungen seitlich aus der Längsachse 94 heraus möglich ist. Das Kugelgelenk 84 ist so ausgebildet, dass sich der Werkzeugträger 76 frei um die Kugel 88 ohne Hinzunahme von irgendwelchen Hilfsmitteln bewegen kann. Am proximalen Ende ist der Schaft 72 mit einem Griff 96 versehen. Die beiden Ausführungsbeispiele von Einstechwerkzeugen 40 und 70 dienen jeweils als "Multi-Mikrofrakturiermeißel", also zum Einbringen von Einstichen in eine Knochenfläche.

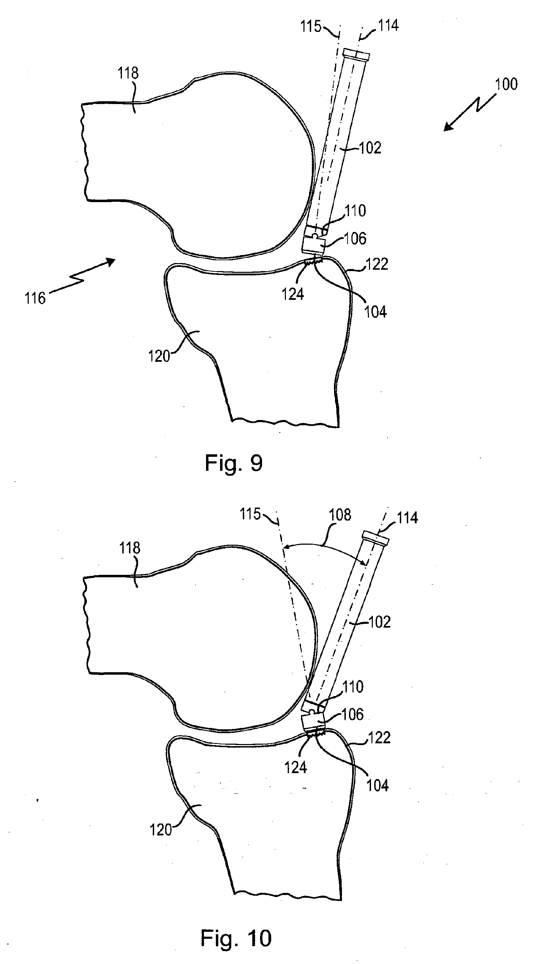

In Fig. 9 und 10 ist ein praktischer Anwendungsfall für solche Einstechwerkzeuge dargestellt, wobei hier beispielhaft ein viertes Ausführungsbeispiel eines Einstechwerkzeuges 100 dargestellt ist, das prinzipiell gleich ausgebildet ist wie das Einstechwerkzeug 10 von Fig. 1 bis 3 mit der einzigen Änderung, dass hier der maximale Schwenkwinkel 108 etwa 30° beträgt.

Demzufolge weist das Einstechwerkzeug 100 einen hohlzylindrischen Schaft 102 auf, der über ein Scharniergelenk mit einem hohlzylindrischen Werkzeugträger 106 verbunden ist, dessen Werkzeug als Stanze 104 ausgebildet ist. Auch hier begrenzt ein Anschlag 110 den maximalen Schwenkwinkel 108 von 30° in einer Richtung aus der Längsachse 114 des Schaftes 102 heraus.

In Fig. 9 ist ein Kniegelenk 116 dargestellt, wobei hier schematisch nur der Oberschenkelknochen 118 und der Unterschenkelknochen 120 dargestellt sind.

An der Knochenhaut 122 des Unterschenkelknochens 120 soll eine Defektstelle 124 repariert werden. Diese Defektstelle 124 ist dadurch entstanden, dass an dieser Stelle durch Reibung mit einer gegenüberliegenden Stelle des Oberschenkelknochens 118 bei normaler Ausrichtung, also bei stehender Position, ein Defekt an der Knochenhaut entstanden ist.

Die Defektstelle 124 wäre mit einer eingangs erwähnten geradlinig ausgerichteten starren Knochenstanze nicht mit optimaler Ausrichtung der Schneide zu erreichen.

In Fig. 9 ist dargestellt, wie die Defektstelle 124 bereits mit leicht schräg angestelltem Schaft 102, der auch schon etwas gegenüber der Längsachse 115 des Werkzeuges 106 angewinkelt ist, angezielt werden kann. Durch die schräge Anstellung trifft die Schneide des Werkzeuges 106 die etwa ebene Defektstelle 124 nur im Bereich eines Umfangpunktes. Dies gibt aber der Handhabungsperson einen Ansatzpunkt oder eine Ansatzstelle.

Durch Abwinkeln des Schaftes 102 gegenüber dem Werkzeugträger 106 kann nunmehr die Schneide so an die Defektstelle 124 gebracht werden, dass sie sich in der zutreffenden Ausrichtung befindet, also die umlaufende Schneidkante komplett auf der Oberfläche der Knochenhaut 122 im Bereich der Defektstelle 124 aufliegt, wie das aus Fig. 10 ersichtlich ist. Nunmehr kann auf den Schaft 102 die notwendige Kraft ausgeübt werden, um die Stanze 104 in den Knochen 120 einzudrücken und dabei ein scheibenförmiges Stück der Knochenhaut 122 um die Defektstelle 124 herum auszustanzen. Auch in dem in Fig. 10 dargestellten maximal abgewinkelten Zustand von 30° gegenüber der Längsachse 114 kann noch eine ausreichende Eindrückkraft ausgeübt werden. Es ist sinnvoll, dabei den maximalen Schwenkwinkel 108 einzunehmen, da dann der Werkzeugträger 106 und das distale Ende des Schaftes 102 über den Anschlag 110 in Berührung stehen und die Eindrückkraft darüber geleitet werden kann. Durch die hohlzylindrischen Körper von Schaft 102 und Werkzeugträger 106 kann nun ein Spatel hindurchgeführt werden, um das ausgestanzte Knochenhautstück zu entnehmen. Anschließend wird das Einstechwerkzeug 100 abgenommen.

Wird gewünscht an der Defektstelle eine Mikrofraktur einzubringen, um aus dem Knochen 120 entsprechende Flüssigkeiten austreten zu lassen, die an dieser Stelle eine neue Knochenhaut bilden, oder um ein einzusetzendes Implantat besser anwachsen zu lassen, kann ein in den Fig. 4 bis 6 oder 7 bis 8 dargestelltes Einstechwerkzeug 40 bzw. 70 angesetzt werden, wobei dann die Größe bzw. der Durchmesser des jeweiligen Werkzeugträgers 46 bzw. 106 so bemessen ist, dass dieser dem Durchmesser des ausgestanzten Bereiches in dem Knochen 120 entspricht. Durch Schlagen auf den Griff 49 oder 96 oder durch kräftiges Eindrücken mit einer Hand, die diese Griffe erfasst hat, kann dann der entsprechend Multi-Mikrofrakturiermeißel 48 bzw. 78 in den Knochen eingeschlagen werden.

In beiden Fällen können mit diesen Werkzeugen aufgrund der schwenkbaren bzw. abwinkelbaren Ausgestaltung des eigentlichen Werkzeuges auch schwer zugängliche Stellen behandelt werden.

## Patentansprüche

1. Medizinisches Einstechwerkzeug zum Eindringen in einen Knochen (120), mit einem eine Längsachse (30, 60, 94, 114) aufweisenden Schaft (12, 42, 72, 102), an dessen distalem Ende (14, 44, 74) ein Werkzeug in Form einer Stanze (104) oder eines Multi-Mikrofrakturiermeißels (48, 78) angeordnet ist, wobei das Werkzeug an einem distalseitigen Werkzeugträger (16, 46, 76, 106) angeordnet ist, **dadurch gekennzeichnet, dass** der distalseitige Werkzeugträger, ohne Zuhilfenahme weiterer Hilfs- oder Steuermittel, frei schwenkbar am distalen Ende des Schaftes (12, 42, 72, 102) angebracht ist, wobei ein Schwenkwinkel (32, 62, 92, 108) der Verschwenkbarkeit des Werkzeugträgers (16, 46, 76, 106) aus der Längsachse (30, 60, 94, 114) heraus durch einen Anschlag (36, 64, 98) auf maximal 45° begrenzt ist.

2. Medizinisches Einstechwerkzeug nach Anspruch 1, **dadurch gekennzeichnet, dass** der Schwenkwinkel (108) durch den Anschlag (98) auf maximal 30° begrenzt ist.

3. Medizinisches Einstechwerkzeug nach Anspruch 1, **dadurch gekennzeichnet, dass** der Schwenkwinkel (32) durch den Anschlag (36) auf maximal 15° begrenzt ist.

4. Medizinisches Einstechwerkzeug nach einem der Ansprüche 1 bis 3, **dadurch gekennzeichnet, dass** der Werkzeugträger (16, 46, 76, 106) eine axiale Länge von maximal 30 mm aufweist.

5. Medizinisches Einstechwerkzeug nach einem der Ansprüche 1 bis 3, **dadurch gekennzeichnet, dass** der Werkzeugträger eine axiale Länge von maximal 20 mm aufweist.

6. Medizinisches Einstechwerkzeug nach einem der Ansprüche 1 bis 3, **dadurch gekennzeichnet, dass** der Werkzeugträger eine axiale Länge von maximal 10 mm aufweist.

7. Medizinisches Einstechwerkzeug nach einem der Ansprüche 1 bis 6, **dadurch gekennzeichnet, dass** der Werkzeugträger (46) um einen sich quer zur Längsachse (60) des Schaftes (42) erstreckenden Achsbolzen (54) frei verschwenkbar ist.

8. Medizinisches Einstechwerkzeug nach einem der Ansprüche 1 bis 6, **dadurch gekennzeichnet, dass** der Werkzeugträger (46) über ein Kugelgelenk (84) in allen Richtungen frei verschwenkbar am Schaft (72) angebracht ist.

9. Medizinisches Einstechwerkzeug nach einem der Ansprüche 1 bis 6, **dadurch gekennzeichnet, dass** der Werkzeugträger (16) über eine Art Scharniergelenk (18) mit einem hohlzylindrischen Schaft (24) verbunden ist und dass der Körper des Werkzeugträgers (16) ebenfalls als Hohlzylinder ausgebildet ist, und dass die Teile Pfanne (26) und Gelenkkopf (28) des Scharniergelenkes (18) jeweils aus der hohlzylindrischen Wand von Schaft (12) und hohlzylindrischem Werkzeugträger (16) herausgearbeitet sind.

10. Medizinisches Einstechwerkzeug nach einem der Ansprüche 1 bis 9, **dadurch gekennzeichnet, dass** der Anschlag (36, 64) eine schräge Anschlagfläche (38, 66) am distalen Ende des Schaftes (12, 42) aufweist.

11. Medizinisches Einstechwerkzeug nach einem der Ansprüche 1 bis 10, **dadurch gekennzeichnet, dass** der Anschlag derart ausgebildet ist, dass eine Verschwenkung aus der Längsachse (30) nur nach einer Seite durchführbar ist.

## Claims

1. Medical puncturing device for penetrating into a bone (120), with a shaft (12, 42, 72, 102) having a longitudinal axis (30, 60, 94, 114), a tool in form of a punch (104) or a multi-fracture chisel (48, 78) is mounted at a distal end (14, 44, 74) of the shaft (12, 42, 72, 102), wherein the tool is arranged at a distal tool carrier (16, 46, 76, 106), **characterized in that** the distal tool carrier is mounted to freely articulate at the distal end of the shaft (12, 42, 72, 102) without any auxiliary or control means, wherein a pivoting angle (32, 62, 92, 108) of the pivoting movement of the tool carrier (16, 46, 76, 106) out of the longitudinal axis (30, 60, 92, 114) is limited to a maximum of 45° by a stop (36, 64, 98).

2. Medical puncturing device of claim 1, **characterized in that** the pivoting angle (108) is limited by the stop (98) to a maximum of 30°.

3. Medical puncturing device of claim 1, **characterized in that** the pivoting angle (32) is limited by the stop (36) to a maximum of 15°.

4. Medical puncturing device of any one of claims 1 through 3, **characterized in that** the tool carrier (16, 46, 76, 106) has an axial length of at most 30 mm.

5. Medical puncturing device of any one of claims 1 through 3, **characterized in that** the tool carrier has an axial length of at most 20 mm.

6. Medical puncturing device of any one of claims 1 through 3, **characterized in that** the tool carrier has an axial length of at most 10 mm.

7. Medical puncturing device of any one of claims 1 through 6, **characterized in that** the tool carrier (46) is mounted via an axle bolt (54) extending transversely to the longitudinal axis (60) of the shaft (42) in the freely pivotable manner.

8. Medical puncturing device of any one of claims 1 through 6, **characterized in that** the tool carrier (46) is mounted via a ball-and-socket joint (84) to freely pivot in all directions at the shaft (72).

9. Medical puncturing device of any one of claims 1 through 6, **characterized in that** the tool carrier (16) is connected to a hollow cylindrical shaft (24) via a kind of hinge (18), and **in that** the body of the tool carrier (16) is shaped as a hollow cylinder too, and **in that** the parts socket (26) and joint head (28) of the hinge (18) are worked out of the hollow cylindrical wall of the shaft (12) and the hollow cylindrical tool carrier (16), respectively.

10. Medical puncturing device of any one of claims 1 through 9, **characterized in that** the stop (36, 64) is an oblique stop surface (38, 66), provided at the distal end of the shaft (12, 42).

11. Medical puncturing device of any one of claims 1 through 10, **characterized in that** the stop is designed in such a way that a pivoting movement out of the longitudinal axis (30) can be formed only to one lateral side.

## Revendications

1. Outil à défoncer médical permettant la pénétration dans un os (120), comportant une tige (12, 42, 72, 102) présentant un axe longitudinal (30, 60, 94, 114), à l'extrémité distale (14, 44, 74) de laquelle est disposé un outil sous la forme d'un poinçon (104) ou d'un multi-microburin à fracturer (48, 78), lequel outil est disposé sur un porte-outil côté distal (16, 46, 76, 106), **caractérisé en ce que** le porte-outil côté distal est monté, sans le secours d'autres moyens auxiliaires ou de commande, librement pivotant à l'extrémité distale de la tige (12, 42, 72, 102), un angle de pivotement (32, 62, 92, 108) de la liberté de pivotement du porte-outil (16, 46, 76, 106) à partir de l'axe longitudinal (30, 60, 94, 114) étant limité à 45° au maximum par une butée (36, 64, 98).

2. Outil à défoncer médical selon la revendication 1, **caractérisé en ce que** l'angle de pivotement (108) est limité à 30° au maximum par la butée (98).

3. Outil à défoncer médical selon la revendication 1, **caractérisé en ce que** l'angle de pivotement (32) est limité à 15° au maximum par la butée (36).

4. Outil à défoncer médical selon l'une des revendications 1 à 3, **caractérisé en ce que** le porte-outil (16, 46, 76, 106) présente une longueur axiale de 30 mm au maximum.

5. Outil à défoncer médical selon l'une des revendications 1 à 3, **caractérisé en ce que** le porte-outil présente une longueur axiale de 20 mm au maximum.

6. Outil à défoncer médical selon l'une des revendications 1 à 3, **caractérisé en ce que** le porte-outil présente une longueur axiale de 10 mm au maximum.

7. Outil à défoncer médical selon l'une des revendications 1 à 6, **caractérisé en ce que** le porte-outil (46) est librement pivotant autour d'un boulon-pivot (54) s'étendant transversalement à l'axe longitudinal (60) de la tige (42).

8. Outil à défoncer médical selon l'une des revendications 1 à 6, **caractérisé en ce que** le porte-outil (46) est monté librement pivotant dans toutes les directions sur la tige (72) par l'intermédiaire d'une articulation à rotule (84).

9. Outil à défoncer médical selon l'une des revendications 1 à 6, **caractérisé en ce que** le porte-outil (16) est relié à une tige cylindrique creuse (24) par une sorte d'articulation à charnière (18) et que le corps du porte-outil (16) est également réalisé sous la forme d'un cylindre creux, et que les parties cuvette (26) et tête d'articulation (28) de l'articulation à charnière (18) sont façonnées respectivement à partir de la paroi cylindrique creuse de la tige (12) et du porte-outil cylindrique creux (16).

10. Outil à défoncer médical selon l'une des revendications 1 à 9, **caractérisé en ce que** la butée (36, 64) présente une surface de butée oblique (38, 66) à l'extrémité distale de la tige (12, 42).

11. Outil à défoncer médical selon l'une des revendications 1 à 10, **caractérisé en ce que** la butée est configurée de façon qu'un pivotement à partir de l'axe longitudinal (30) ne soit réalisable que vers un côté.
